(19) Europäisches Patentamt
European Patent Office
Office européen des brevets

(11) **EP 3 465 506 B1**

(12) **EUROPEAN PATENT SPECIFICATION**

(45) Date of publication and mention
of the grant of the patent:
**03.04.2024 Bulletin 2024/14**

(21) Application number: **17729697.7**

(22) Date of filing: **01.06.2017**

(51) International Patent Classification (IPC):
*G16B 20/20* $^{(2019.01)}$     *G16B 50/10* $^{(2019.01)}$
*G16B 50/30* $^{(2019.01)}$     *G16H 70/60* $^{(2018.01)}$

(52) Cooperative Patent Classification (CPC):
**G16B 20/20; G16B 50/10; G16B 50/30;
G16H 70/60**

(86) International application number:
**PCT/US2017/035466**

(87) International publication number:
**WO 2017/210437 (07.12.2017 Gazette 2017/49)**

(54) **METHODS AND SYSTEMS FOR DESIGNING GENE PANELS**

VERFAHREN UND SYSTEME ZUM ENTWERFEN VON GENPANELS

PROCÉDÉS ET SYSTÈMES DESTINÉS À LA CONCEPTION DE PANNEAU GÉNÉTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.06.2016 US 201662344078 P
16.09.2016 US 201662395828 P
23.05.2017 US 201762509860 P
25.05.2017 US 201762510906 P**

(43) Date of publication of application:
**10.04.2019 Bulletin 2019/15**

(73) Proprietor: **Life Technologies Corporation
Carlsbad, California 92008 (US)**

(72) Inventors:
• **WILLIAMS, Emily
Carlsbad
California 92008 (US)**

• **TIAN, Yuan
Carlsbad
California 92008 (US)**
• **ZHU, Yun
Carlsbad
California 92008 (US)**
• **SHTIR, Corina
Carlsbad
California 92008 (US)**

(74) Representative: **Ziermann, Oliver
Life Technologies GmbH
Frankfurter Straße 129b
64293 Darmstadt (DE)**

(56) References cited:
**WO-A1-2011/151500     WO-A1-2015/051275
WO-A2-2014/037914     US-A1- 2011 183 336
US-A1- 2015 154 354     US-B2- 7 203 698**

**Description**

**SUMMARY**

Next-generation sequencing (NGS) technologies continue to be deployed in clinical laboratories, enabling rapid transformations in genomic medicine. Particularly, targeted sequencing is preferred as it allows users to focus time, expenses, and data analysis on specific regions of interest. Targeted next-generation sequencing panels enable interrogation of multiple genes across many samples to more deeply understand human genetic disease. However, finding all relevant genes, developing robust, high performing multiplex panels, and implementing scalable, reproducible and accurate analysis pipelines is challenging. A critical challenge is how to effectively prioritize genes and regions for selected diseases, which by conventional approaches requires tremendous expert efforts. There is a need for an informative bioinformatics engine to automate the gene selection process for gene panel design.

This need is addressed by the subject matter of the independent claims. Advantageous embodiments are provided in the dependent claims.

In accordance with the present teachings, there is provided a non-transitory machine-readable medium or article as defined in claim 1.

Further in accordance with the present teachings, there is a computer-implemented method of selecting genes for a gene panel as defined in claim 2.

Also, in accordance with the present teachings, there is provided a combination of a kit as defined in claim 16 comprising a set of primers associated with a set of genes in a gene panel, the set of genes selected for the gene panel by the method of any of the claims 2-15, and a non-transitory machine-readable medium or article according to claim 1.

**BRIEF DESCRIPTION OF THE DRAWINGS**

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

FIG. 1 is a block diagram showing a disease association database, gene scoring algorithm and virtual panel library for identifying and ranking gene-disease associations in an example of an embodiment.
FIG. 2 illustrates an example of a query and a response of the graph database system for the disease association database.
FIG. 3 is an example of network graph representing associations of diseases with genes and phenotypes resulting from information stored in the disease association database.
FIG. 4 is a diagram of an example of gene-disease associations at a level of a disease hierarchy.
FIG. 5 is a diagram of an example of gene-disease associations and hierarchical disease associations between multiple levels of a disease hierarchy.
FIG. 6 is an example of tables of ranked genes for diseases at various levels of the disease hierarchy using UMLS disease identifiers.
FIG. 7 is an example of tables of ranked genes for diseases at various levels of the disease hierarchy using MeSH disease identifiers.
FIG. 8 is an example of a density function of rank weighted sum scores.
FIG. 9 is block diagram of gene cluster processing for the virtual panel library, in an example of an embodiment.
FIG. 10 is a table of exemplary results of annotated gene clusters with level-1 disease classifications.
FIG. 11 illustrates a system for designing primers or assays in accordance with the present teachings.

**DETAILED DESCRIPTION**

In accordance with the teachings and principles embodied in this application, new methods, systems, kits and computer readable media for selecting genes for targeted next-generation sequencing panels.

In this application, "amplifying" generally refers to performing an amplification reaction.

In this application, "amplicon" generally refers to a product of a polynucleotide amplification reaction, which includes a clonal population of polynucleotides, which may be single stranded or double stranded and which may be replicated from one or more starting sequences. The one or more starting sequences may be one or more copies of the same sequence, or they may be a mixture of different sequences that contain a common region that is amplified such as, for example, a specific exon sequence present in a mixture of DNA fragments extracted from a sample. Preferably, amplicons may be formed by the amplification of a single starting sequence. Amplicons may be produced by a variety of amplification reactions whose products comprise replicates of one or more starting, or target, nucleic acids. Amplification reactions producing amplicons may be "template-driven" in that base pairing of reactants, either nucleotides or oligonucleotides, have complements in a template polynucleotide that are required for the creation of reaction products. Template-driven reactions may be primer extensions with a nucleic acid polymerase or oligonucleotide ligations with a nucleic acid ligase. Such reactions include, for example, polymerase chain reactions (PCRs), linear polymerase reactions, nucleic acid sequence-based amplifications (NASBAs), rolling circle amplifications, for example, including such reactions disclosed in the following references: Gelfand et al., U.S. Pat. No. 5,210,015; Kacian et al., U.S. Pat. No. 5,399,491; Mullis, U.S. Pat. No. 4,683,202; Mullis et al., U.S. Pat. Nos. 4,683,195; 4,965,188; and 4,800,159; Lizardi, U.S. Pat. No. 5,854,033; and Wittwer et al., U.S. Pat. No. 6,174,670. In an exemplary embodiment, amplicons may be produced by PCRs. Amplicons may also be generated using rolling circle amplification to form a single body that may exclusively occupy a microwell as disclosed in Drmanac et al., U.S. Pat. Appl. Publ. No. 2009/0137404.

In this application, "primer" generally refers to an oligonucleotide, either natural or synthetic, that is capable, upon forming a duplex with a polynucleotide template, of acting as a point of initiation of nucleic acid synthesis and being extended from its 3' end along the template so that an extended duplex may be formed. Extension of a primer may be carried out with a nucleic acid polymerase, such as a DNA or RNA polymerase. The sequence of nucleotides added in the extension process may be determined by the sequence of the template polynucleotide. Primers may have a length in the range of from 14 to 40 nucleotides, or in the range of from 18 to 36 nucleotides, for example, or from N to M nucleotides where N is an integer larger than 18 and M is an integer larger than N and smaller than 36, for example. Other lengths are of course possible.

In this application, "oligonucleotide" generally refers to a linear polymer of nucleotide monomers and may be DNA or RNA. Monomers making up polynucleotides are capable of specifically binding to a natural polynucleotide by way of a regular pattern of monomer-to-monomer interactions, such as Watson-Crick type of base pairing, base stacking, Hoogsteen or reverse Hoogsteen types of base pairing, for example. Such monomers and their internucleosidic linkages may be naturally occurring or may be analogs thereof, e.g., naturally occurring or non-naturally occurring analogs. Non-naturally occurring analogs may include PNAs, phosphorothioate internucleosidic linkages, bases containing linking groups permitting the attachment of labels, such as fluorophores, or haptens, for example. In an exemplary embodiment, oligonucleotide may refer to smaller polynucleotides, for example, having 5 - 40 monomeric units. Polynucleotides may include the natural deoxyribonucleosides (e.g., deoxyadenosine, deoxycytidine, deoxyguanosine, and deoxythymidine for DNA or their ribose counterparts for RNA) linked by phosphodiester linkages. However, they may also include non-natural nucleotide analogs, e.g., including modified bases, sugars, or internucleosidic linkages. In an exemplary embodiment, a polynucleotide may be represented by a sequence of letters (upper or lower case), such as "ATGCCTG," and it will be understood that the nucleotides are in 5' → 3' order from left to right and that "A" denotes deoxyadenosine, "C" denotes deoxycytidine, "G" denotes deoxyguanosine, and "T" denotes deoxythymidine, and that "I" denotes deoxyinosine, and "U" denotes deoxyuridine, unless otherwise indicated or obvious from context.

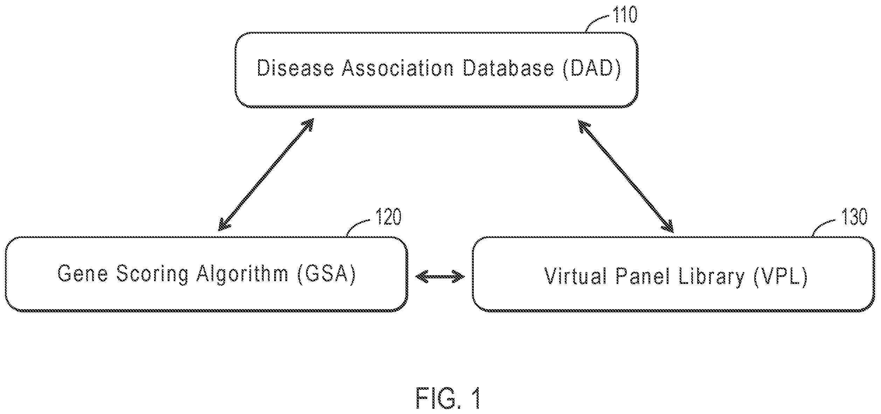

In accordance with the present teachings, a system for identifying and ranking gene-disease associations for gene panel design may include one or more modules of a disease association database (DAD) module 110, a gene scoring algorithm (GSA) module 120 and a virtual panel library (VPL) module 130, as shown in FIG. 1. The DAD module 110 organizes and stores information on associations between genes and diseases, including a hierarchical organization of diseases. The GSA module 120 applies gene scoring and ranking algorithms using information on gene-disease associations and disease hierarchies retrieved from the DAD module 110. The VPL module 130 analyzes the rank scores from the GSA module 120 to cluster genes having similar disease association patterns and associate the gene clusters with diseases.

In some embodiments, the disease association database (DAD) module 110 comprises a graph database system that is configured to store disease information, phenotype information, gene information and association information, including associations between diseases, disease-phenotype associations and gene-disease associations. The disease association information may include disease hierarchy information. The gene-disease association information may include a strength parameter for each gene-disease association.

The graph database architecture has advantages over standard relational database architectures for representing highly interconnected data. The graph database architecture emphasizes the relationships between data points. The emphasis on relationships is advantageous for representing disease hierarchies and gene-disease associations. Graph databases enable rapid and intuitive queries of the relationships between data points. In contrast, for a standard relational database queries of associations would be slow and tedious to design.

In some embodiments, the data structures of the DAD module 110 include nodes and edges, where an edge is associated with two nodes in the graph data base architecture. Disease information is stored in a node assigned to the disease, or a disease node. The disease node stores disease information, including a disease identifier and the name of the disease. The disease node may store additional information, such as the source of the disease information. Hierarchical relationships between diseases are represented by edges. For example, when two diseases have a parent-child relationship in the disease hierarchy, the edge associating the two disease nodes may store attributes including the disease identifiers for the diseases and a direction attribute from parent disease to child disease.

In some embodiments, the DAD module 110 includes nodes assigned to store gene information, or gene nodes. The gene node stores gene information, including a gene identifier, gene name and gene symbol. A disease node may be linked to a gene node by an edge indicating the gene-disease association. The edge may store attributes for a gene-disease association, including the disease identifier, the gene identifier and a strength parameter for the gene-disease association.

In some embodiments, the DAD module 110 includes nodes assigned to store phenotype information, or phenotype nodes. The phenotype node stores phenotype information, including a phenotype identifier. A phenotype node may be linked to a disease node by an edge indicating a phenotype-disease association. The edge associating the phenotype node and disease node stores attributes, including the phenotype identifier and the disease identifier. The edge may store additional information, such as the source of the phenotype-disease association.

In some embodiments, the disease information, including the disease identifier, disease name and disease hierarchy information stored in the DAD module 110 are based on the Unified Medical Language System (UMLS). The UMLS incorporates a number of controlled vocabularies, including Medical Subject Headings, or MeSH. For example, the UMLS disease identifier is C0004615 for bacterial infections and mycoses. For example, the MeSH disease identifier is D001523 for mental disorders. UMLS is described by Bodenreider, "The Unified Medical Language System (UMLS): integrating biomedical terminology," Nucleic Acids Research, Vol. 32, Database issue, pp. D267-D270 (2004).

In some embodiments, the gene-disease associations stored in the DAD module 110 are based on DisGeNET, which scores gene-disease associations according to expert-curated sources (e.g. CTD, CLINVAR, and ORPHANET), predicted data using mouse models, and text-mining of publications. The DisGeNET score was developed to rank the gene-disease associations according to their level of evidence. DisGeNET gene-disease association score takes into account the number and type of sources (level of curation, model organisms) and the number of publications supporting the association. The score values range from 0 to 1. According to DisGeNet, a score of 0.1 corresponds to an average of about 3 sources of evidence. A score of 0.25 corresponds to between 4 and 5 sources of evidence for single gene-disease assocation. DisGeNET is described by Piñero et al., "DisGeNET: a discovery platform for the dynamical exploration of human diseases and their genes," Database, Vol. 2015, Article ID bav028, pp. 1-17 (2015).

In some embodiments, the MeSH disease hierarchies are combined with DisGeNET gene-disease associations in the DAD module 110. MeSH provides information for a mathematical graph indicating disease parent/child relationships; e.g., "autoimmune disease" is a parent disease of "rheumatoid arthritis", while "juvenile rheumatoid arthritis" is a child disease of "rheumatoid arthritis". The nodes in the graph, representing the diseases, are related to nodes representing genes by edges having a strength parameters. The strength parameters are based on a score provided by DisGeNET for each gene-disease pair. The DisGeNET score is a number between 0 and 1. Taken together, the disease hierarchy combined with the gene-disease associations provide a way to look up genes involved with any disease or group of diseases at any level in the disease hierarchy.

In some embodiments, the graph database architecture of the DAD module 110 may be implemented in whole or in part on Neo4j graph database. A database implemented using Neo4j can be accessed using the Cypher Query Language.

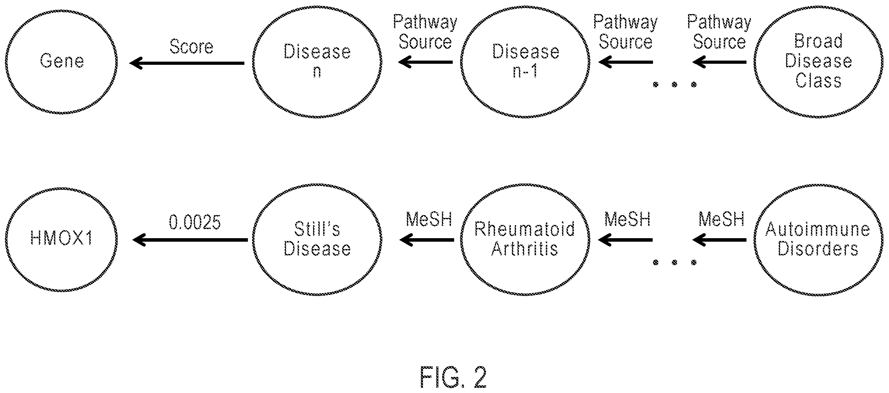

FIG. 2 illustrates an example of a query and a response of the graph database system for the DAD module 110. In this example, a query may define patterns for tracing paths in the network, such as illustrated by the top row of FIG. 2. The query may return information for pathways and nodes, as illustrated in the bottom row of FIG. 2. In this example, the edges for disease associations store source information. The response indicates the source for disease associations is MeSH. In this example, the edge for gene-disease association stores the strength parameter, or score obtained from DisGeNET, which is 0.0025.

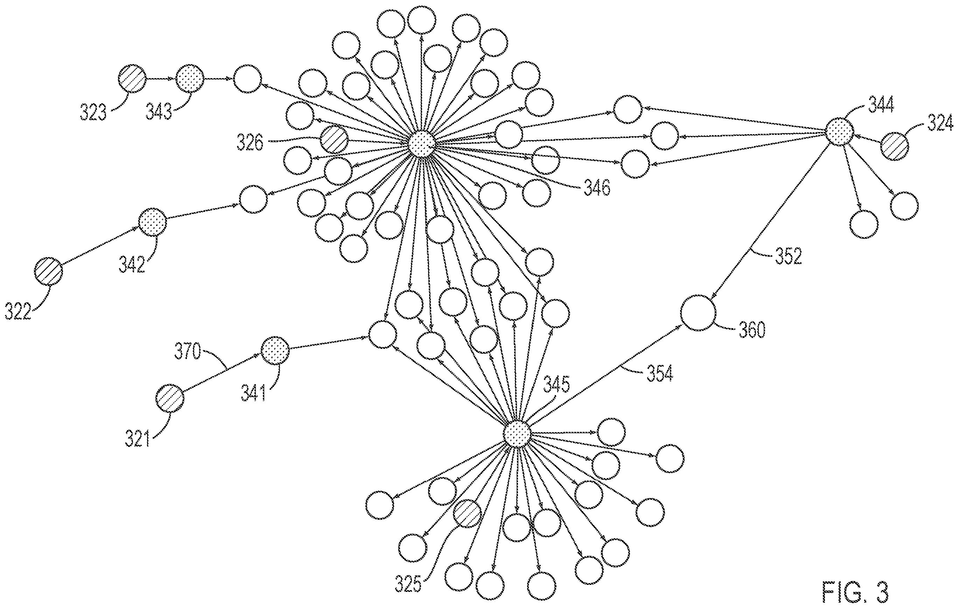

FIG. 3 is an example of network graph representing associations of diseases with genes and phenotypes resulting from a query of the DAD module 110. The graph shows associations among disease nodes, 341, 342, 343, 344, 345 and 346 (spotted circles) and phenotype nodes 321, 322, 323, 324, 325 and 326 (striped circles). For example, the edge 370 associating disease node 341 and phenotype node 321 may include the UMLS concept information. The clear circles represent various gene nodes associated with the various disease nodes. This example shows several gene nodes that have gene-disease associations with multiple disease nodes. For example, gene node 360 and disease node 344 have a gene-disease association represented by edge 352. Gene node 360 also has a gene-disease association with disease node 345, represented by edge 354. Edges 352, 354 and all the edges connecting the disease nodes and gene nodes store the strength parameter for corresponding gene-disease association. For example, the strength parameter may use the DisGeNET score.

In some embodiments, the DAD module 110 may be adapted to store information mined from any scientific source database that can contribute to the disease information, gene information and gene-disease association information. The DAD module 110 may be updated as releases of source databases evolve and include new information.

In some embodiments, the gene scoring algorithm (GSA) module 120 ranks genes by their relevance to a disease in the disease hierarchy using information retrieved from the DAD module 110. The GSA module 120 uses the gene-disease association strength parameters, such as DisGeNET scores, and disease hierarchy information from the DAD module 110 and applies a scoring method to prioritize genes for a specific disease of interest. The GSA module 120 can produce a list of ranked genes for one or more diseases at any level of the disease hierarchy.

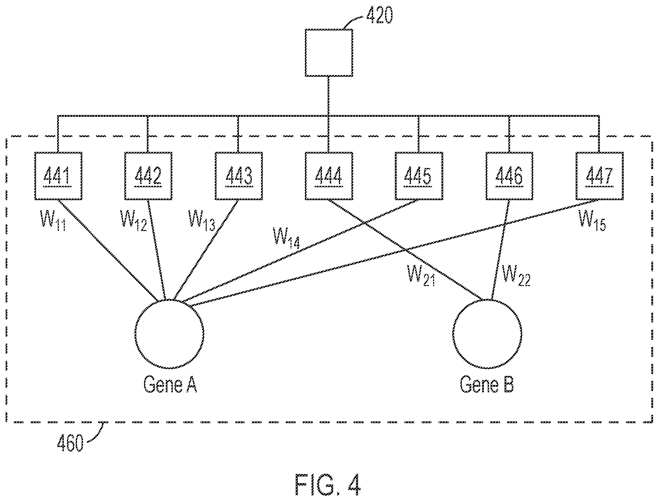

FIG. 4 is a diagram of an example of gene-disease associations at a level of a disease hierarchy. In this example, Gene A and Gene B represent two genes that have gene-disease associations at a level 460 of the disease hierarchy. Gene A has gene-disease associations with diseases 441, 442, 443, 445 and 447 with respective strength parameters $w_{11}$, $w_{12}$, $w_{13}$, $w_{14}$, and $w_{15}$. Gene B has gene-disease associations with diseases 444 and 446 with respective strength parameters $w_{21}$ and $w_{22}$. The disease of interest 420 is one level higher in the disease hierarchy and has a parent-child relationship with the diseases 441 to 447 at the lower level 460. In some embodiments, the GSA module 120 can apply a scoring method to the strength parameters $w_{ij}$ associated with Genes A and B at the lower level 460 of the disease hierarchy to rank them with respect to the disease of interest 420.

In some embodiments, the scoring method may be a simple sum of the strength parameters $w_{ij}$ for the gene-disease associations of the $i^{th}$ gene, or an average of the strength parameters. Such a score is advantageous because it considers all diseases associated with the gene. However, such a score may be disadvantageous because a number of diseases having small strength parameters may add up to a relatively large score.

In some embodiments, the scoring method may simply select a maximum strength parameter, such as score $= \max_j (w_{ij})$ for the gene-disease associations of the $i^{th}$ gene. Such a score may be advantageous because a gene strongly associated with one disease at a given level of the hierarchy would be included. However, a score based on the maximum may be disadvantageous because other diseases associated with the $i^{th}$ gene that may have smaller, but not insignificant strength parameters, are left out.

In some embodiments, the scoring method includes calculating a rank-weighted sum score (RWSS). The RWSS can be calculated as follows for the $i^{th}$ gene associated with diseases at a given level:

   1) Apply a weight to each strength parameter of the gene-disease association of the $i^{th}$ gene to form weighted strength parameters, and

   2) Sum the weighted strength parameters to produce a rank score for the $i^{th}$ gene.

In some embodiments, the GSA module 120 may calculate the rank scores for all the genes having a gene-disease association with diseases at the given level of the disease hierarchy.

In some embodiments, the GSA module 120 may determine the weights as follows:

   1) Determine an order index k for each strength parameter for an $i^{th}$ gene based on the order of strength parameter values from a highest value (order index k = 1) to a lowest value (order index k = n) for the $i^{th}$ gene's associations with n diseases, and
   2) Set the weight for each strength parameter to a function of the inverse of the order index, f(1/k).

An example of f(1/k) is $f(1/k^t)$, where t is a positive real number. In various examples for an $i^{th}$ gene, where t=0.5 the weight is $1/k^{0.5}$ and rank $\text{score}_i = \sum_k w_{ik}/\sqrt{k}$ ; where t=1 the weight is 1/k and rank score$_i = \Sigma_k w_{ik}/k$; where t=2, the weight is $1/k^2$ and rank score; $= \Sigma_k w_{ik}/k^2$, and so on for other possible values of t. Preferably, t=1 and the rank score for the $i^{th}$ gene is calculated by:

$$\text{rank score}_i = \sum_k w_{ik}/k \qquad 1 \leq k \leq n \qquad (1)$$

In some embodiments, the GSA module 120 ranks the genes based on the rank scores determined using equation (1) for genes associated with diseases at the given level of the disease hierarchy. For example, the genes may be ranked from highest rank score to lowest rank score to form a table of ranked genes. The genes may be listed in the table in rank order from the gene with highest rank score to the gene with the lowest rank score. Each gene may be assigned a rank order index, an integer indicating the order of the rank scores, where the gene with the highest rank score is assigned a rank order index of 1. The resulting table of ranked gene information may be linked to a disease at a higher level than the given level in the disease hierarchy, where there is a hierarchical relationship between diseases at the given level and the disease at the higher level.

Returning to the example of FIG. 4, the GSA module 120 would perform the following:

   1) Calculate the rank scores for Gene A and Gene B using equation (1) to produce rank score$_A$ and rank scores,

2) Rank Genes A and B based on rank score$_A$ and rank scores to form a table of ranked gene information, and

3) Link the rank scores and ranked gene information to the disease of interest 420.

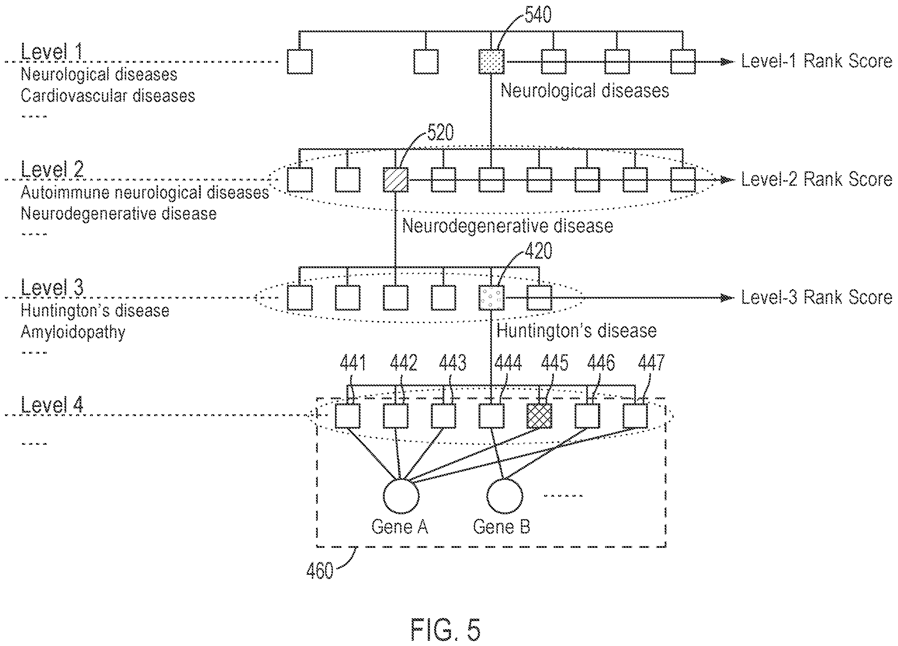

FIG. 5 is a diagram of an example of gene-disease associations and hierarchical disease associations between multiple levels of a disease hierarchy. FIG. 5 illustrates additional hierarchical associations of diseases above those illustrated in FIG. 4. The exemplary number of levels and description of levels are for illustrative purposes and are not limiting. Levels 1 through 4 represent hierarchical levels in the disease hierarchy from broad to specific, such as the disease hierarchy information stored in the DAD module 110. Level 1, the top level, includes the broadest disease classifications, such as neurological diseases and cardiovascular diseases. The neurological disease node 540 is the parent to the diseases represented at level 2. The neurodegenerative disease node 520 is parent to all the diseases represented at level 3. Level 3 includes the disease of interest 420 from FIG. 4, which for this example is Huntington's disease. The Huntington's disease node (disease of interest 420) is the parent to all the diseases represented at level 4 (level 460). For example, the rank scores and ranked gene information described above with respect to FIG. 4 is linked with disease of interest 420, or Huntington's disease, at level 3 in FIG. 5. For FIG. 5, the rank scores and ranked gene information may be linked with diseases in higher levels of the disease hierarchy, including neurodegenerative diseases (node 520) at Level 2 and neurological diseases (node 540) at Level 1 which have hierarchical relationships with Huntington's disease.

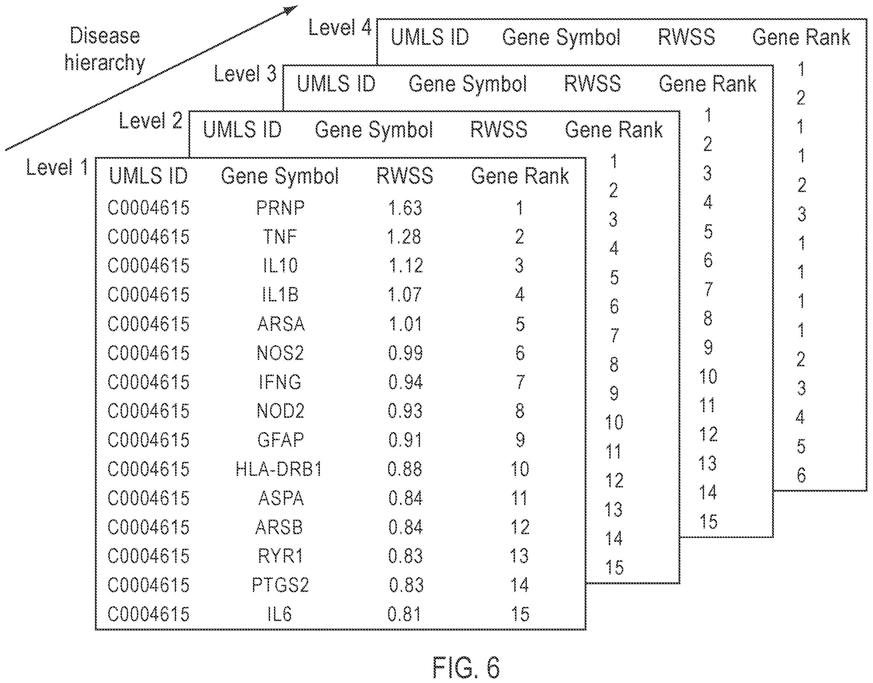

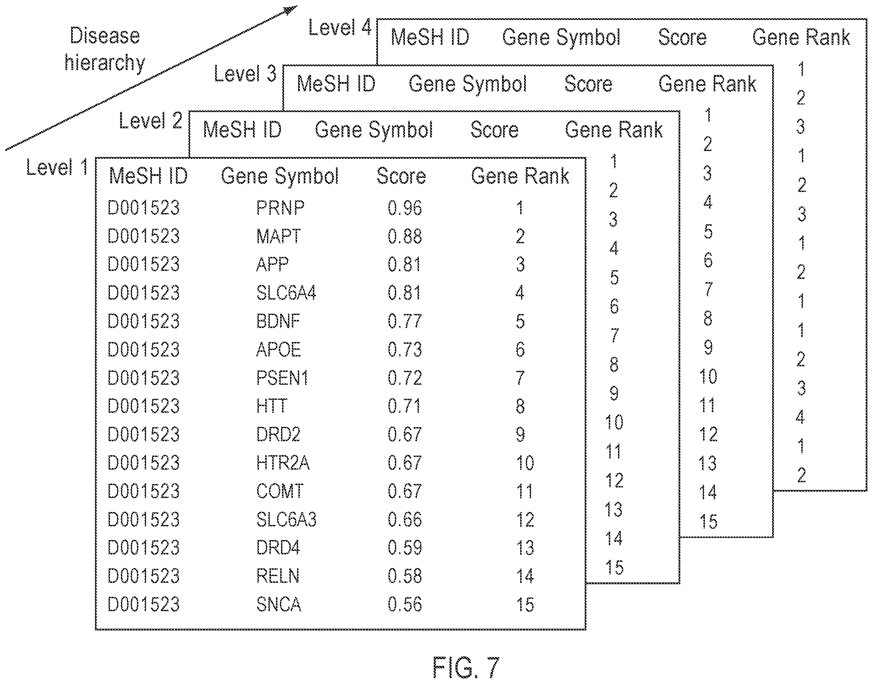

FIG. 6 is an example of tables of ranked genes for diseases at various levels of the disease hierarchy using UMLS disease identification. In FIG. 6, the tables include the UMLS ID for the disease identifier, gene symbols, the scores and the gene rank order indices. FIG. 7 is an example of tables of ranked genes for diseases at various levels of the disease hierarchy using MeSH disease identification. In FIG. 7, the tables include the MeSH ID for the disease identifier, gene symbols, the scores and the gene rank order indices. In FIG. 6 and FIG. 7, the tables show the genes listed in rank order from highest to lowest rank score. For Levels 1-3, the tables include scores that are rank scores determined using rank-weighted sum scores as described above. For Level 4, at the lowest level of the hierarchy for this example, the gene ranks reflect the order of the strength parameters $w_{ij}$, such as the DisGeNET scores.

In some embodiments, the GSA module 120 applies a threshold to the rank scores prior to ranking the genes based on rank scores. The GSA module 120 selects rank scores with values greater than or equal to the threshold and ranks those genes having the selected rank scores. Applying the threshold has advantages of reducing computations for ranking genes having below-threshold rank scores and reducing memory size or storage requirements for the tables of ranked genes generated for the levels of the disease hierarchy.

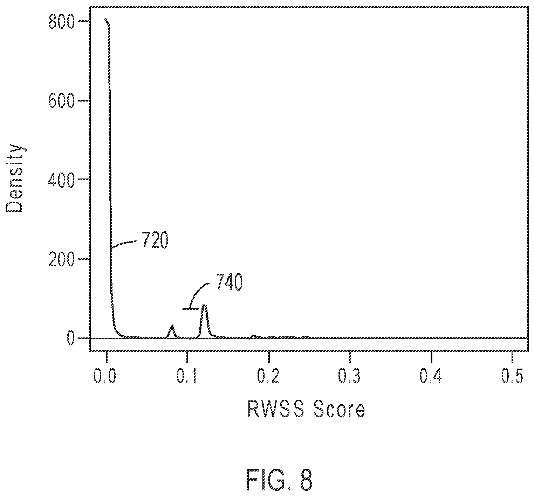

FIG. 8 is an example of a density function 720 of numbers of gene-disease pairs having of rank weighted sum scores along the x-axis. The density function 720 was calculated for 831,405 input RWSS scores using the R programming language for statistical computing. The density function shows that a large portion of the RWSS scores are less than 0.05. Setting the threshold in the interval 740 eliminates a substantial portion of low rank scores. Table 1 shows examples of results after applying different thresholds to the rank scores calculated according to equation (1). The initial number of input rank scores for gene-disease pairs is 831,405. The number of gene-disease pairs remaining after thresholding the ranks scores is shown in Table 1's right column. The threshold values T where $0.09 \leq T \leq 0.11$, corresponding to the interval 740 in FIG. 8, produce similar numbers of gene-disease pairs for the rank scores remaining after the thresholding. The similar numbers of gene-disease pairs indicates stability for gene ranking results when the threshold is set in the interval $0.09 \leq T \leq 0.11$.

Table 1.

| Threshold | Number of gene-disease pairs |
| --- | --- |
| 0.05 | 135,906 |
| 0.08 | 124, 092 |
| 0.09 | 113,451 |
| 0.1 | 112,676 |
| 0.11 | 112,342 |
| 0.12 | 79,331 |
| 0.13 | 38,846 |

In some embodiments, applying a threshold to the rank scores prior to ranking the genes can produce substantial improvements in both compute efficiency and memory/storage efficiency. For example, referring to Table 1, by applying a threshold value of 0.1 to the rank scores, the number of rank scores used for ranking the genes of the gene-disease pairs is reduced from 831,405 to 112,676. The number of genes to be ranked is reduced, thus reducing the computational burden. The sizes of tables of ranked genes associated with diseases at the different levels of the disease hierarchy are also reduced, providing savings in the memory and storage requirements.

In some embodiments, the rank scores are calculated according to equation (1), where the weights applied to the strength parameters $w_{ij}$ are the inverse 1/k of the order index k. In some embodiments, the threshold value applied to the rank scores may be in the range of about 0.09-0.11, or about 0.095-0.105, or about 0.08-0.12, or about 0.05 to 0.13, or about 0.05-0.0.09, 0.08-0.09, or about 0.90-0.10, or about 0.10-0.11, or about 0.11 to 0.12, or about 0.12-0.13, or a subinterval of one of these ranges.

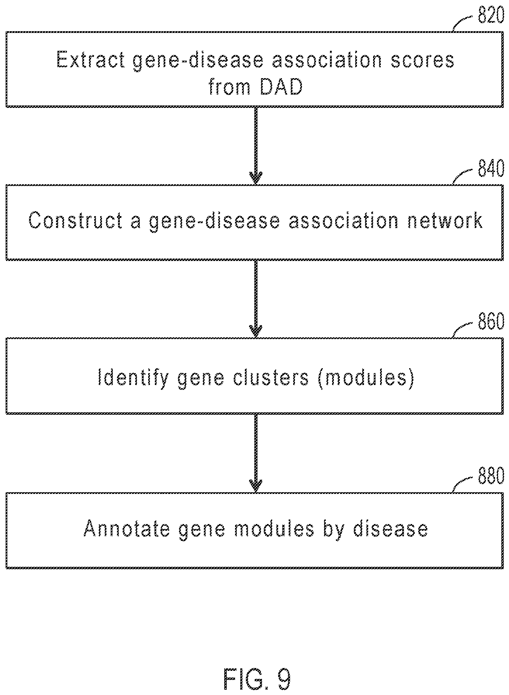

In some embodiments, the virtual panel library (VPL) module 130 analyzes the rank scores for diseases at a level of the disease hierarchy to cluster genes having similar disease association patterns and associate the gene clusters with diseases. FIG. 9 is an exemplary block diagram of gene cluster processing for the VPL module 130. At step 820, the gene-disease association scores, such as a table of the rank scores associated with level-1 diseases, are retrieved from the DAD module 110. Level 1 indicates the top level of the disease hierarchy and includes broad disease classifications. The table may comprise a matrix, where a row indicates a level-1 disease and a column indicates a gene, such that the each column vector contains the gene's rank scores for each of the level-1 diseases. At step 840, constructing a gene-disease association network includes cross-correlating the column vectors of rank scores corresponding to the genes to generate a correlation matrix. Each element of the correlation matrix is a cross-correlation value (or dot product) of two column vectors of rank scores for two genes. The order of the rows and columns of the correlation matrix may be shifted so that high correlation values are grouped towards the diagonal of the correlation matrix. At step 860, thresholding is applied to the correlation values to select gene clusters, or gene modules, of highly correlated genes. At step 880, a principal component analysis of each gene module associates the gene module with a disease. For each identified gene module, a principal component analysis is applied to a matrix comprising the column vectors of rank scores corresponding to each gene in the gene module to generate a principal component vector. Determining the element of the principal component vector having the maximum value indicates the disease associated with the genes in the gene module. The correlations and p-values of the principal component vector for each gene module and each level-1 disease may be calculated to give an association score for the gene module and the disease. The indicated disease may be annotated for the group of genes of the gene module. For example, steps 840, 860 and 880 may be implemented using weighted correlation network analysis (WGCNA), as described in Langfelder et al., "WGCNA: an R package for weighted correlation network analysis," BMC Bioinformatics, Vol. 9:559, pp. 1-13 (2008).

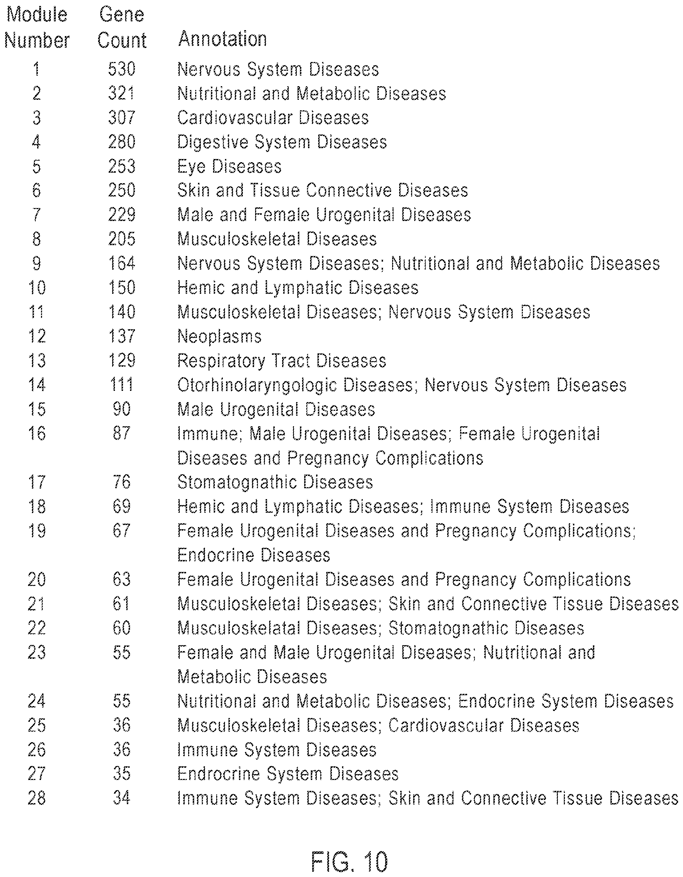

FIG. 10 is a table of exemplary results of annotated gene clusters with level-1 disease classifications. Association patterns for 4,000 genes selected for a clinical exome with 16 high-level MeSH categories relevant to inherited diseases were analyzed. The results were obtained by applying the steps of gene cluster processing for the VPL module 130, as described with respect to FIG. 9. For this example, 28 gene modules were identified by step to identify gene clusters (modules) 860. The number of genes in each module is indicated in the GeneCount column. The Annotation column lists which of the 16 level-1 diseases were identified for the gene module based on the principal component analysis of the rank scores for the genes of the gene modules, as described for step to annotate gene modules by disease 880. While this example shows results for associating genes with level-1 disease classifications, the gene cluster processing for the VPL module 130 described above can be applied to investigate gene-disease associations at any level of the disease hierarchy.

Leamon et al., U.S. Pat. Appl. Publ. No. 2010/0295819 will be referred to as the '819 application. In accordance with the teachings and principles embodied in the '819 application, new methods, computer readable media, and systems are provided that identify or design products or kits that use PCR to enrich one or more genomic regions or targets of interest for subsequent sequencing and/or that include primers or assays that maximize coverage of one or more genomic regions or targets of interest while minimizing one or more of off-target hybridization, a number of primers, and a number of primer pools.

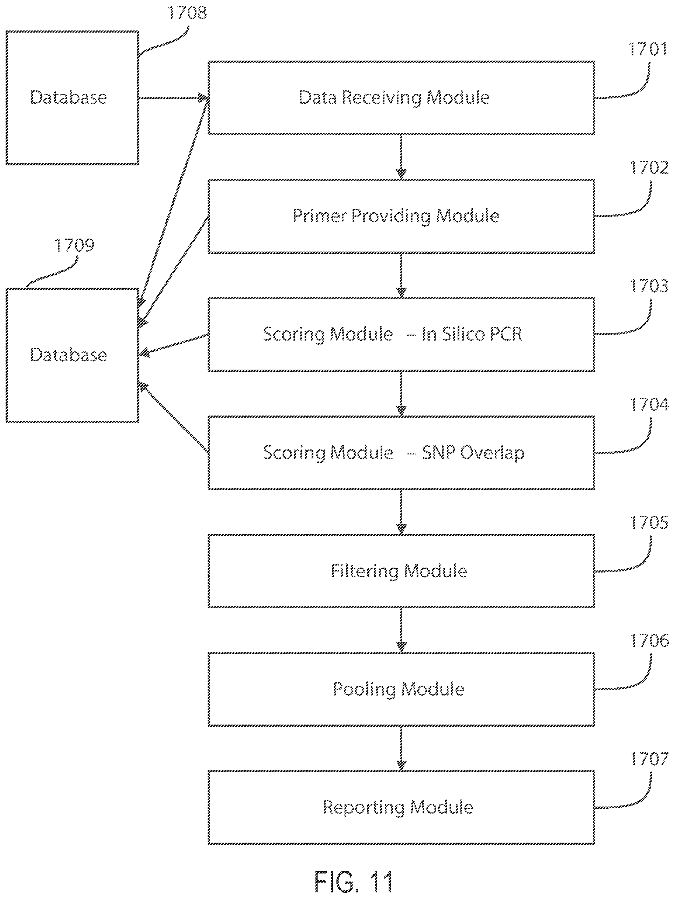

FIG. 11 (FIG. 17 in the '819 application) illustrates a system for designing primers or assays according to the present teachings. The system includes a data receiving module 1701, a primer providing module 1702, a scoring (in silico PCR) module 1703, a scoring (SNP overlap) module 1704, a filtering module 1705, a pooling module 1706, and a reporting module 1707. The system also includes a database 1708, which may include data regarding genetic annotations, SNP-related data, or other genetic data such as identification of a repeat, chromosome, position, direction, etc., for example, or any other type of information that could be related to a genomic region or target of interest, and a database 1709, which may include primer-related data such as a melting temperature (Tm), a chromosome, a position, a direction, and SNP overlap information, etc., for example, or any other type of information that could be related to primers. The system may be implemented in or using one or more computers and/or servers using one or more software components, which may not be accessible or released to customers who may be ordering custom primers or assays that may be designed using such a system. Customers may order custom primers or assays at least in part through a web-accessible data portal by providing one or more genomic regions or targets of interest in any suitable format. In an exemplary embodiment, there is provided a method performing steps including the general steps associated with modules 1701-1707 and databases 1708 and 1709 (e.g., receiving data, providing primers, scoring primers and/or amplicons, filtering primers and/or amplicons, pooling primers and/or amplicons, reporting results, and querying databases).

In some embodiments, GSA module 120 may provide the ranked gene information for gene-disease associations to the database 1708. In some embodiments, the VPL module 130 may provide the annotated gene cluster with disease classification information to the database 1708. In some embodiments, the DAD module 110 may provide the disease association database information to the database 1708.

According to an exemplary embodiment, there is provided a system, including: (1) a disease association database stored in memory and a processor communicatively connected with the memory. The disease association database is configured to store disease information for a plurality of diseases and gene information for a plurality of genes. The disease association database includes disease associations between diseases in the plurality of diseases and gene-disease associations between the diseases and associated genes in the plurality of genes. The disease associations include a disease hierarchy and the gene-disease associations include a strength parameter for each gene-disease association. The processor configured to: (1) retrieve gene-disease associations of genes associated with diseases at a given level in the disease hierarchy from the disease association database, wherein the diseases at the given level have hierarchical relationships with a given disease at a higher level in the disease hierarchy; (2) for each gene associated with the diseases at the given level, apply a weight to the strength parameter for each gene-disease association; (3) add the weighted strength parameters of the gene-disease associations to form a rank score for the each gene associated with the diseases at the given level; and (4) rank the genes associated with the diseases at the given level based on the rank scores to provide ranked gene information associated with the given disease at the higher level for a table of ranked genes. The processor may be further configured to apply a threshold to the rank scores and to rank the genes having rank scores greater than or equal to the threshold. The disease association database may comprise a graph database system having a plurality of nodes and a plurality of edges, wherein an edge is associated with two nodes. A disease node of the plurality of nodes may store the disease information for one of the plurality of diseases. The disease information at the disease node may include a disease identifier. An edge associating two disease nodes may represent a hierarchical relationship of the two diseases. A gene node of the plurality of nodes may store the gene information for one of the plurality of genes. The gene information stored at the gene node may include a gene identifier. A disease node of the plurality of nodes and a gene node of the plurality of nodes are associated by a gene-disease edge, wherein the gene-disease edge stores the strength parameter for the gene-disease association. The disease association database may further include phenotype information for a plurality of phenotypes and phenotype-disease associations between the diseases and associated phenotypes in the plurality of phenotypes. The processor may be further configured to respond to a query to the disease association database to provide the gene-disease associations for a graphical display. The processor may be further configured to respond to a selection of the given disease by a user to select one or more of the ranked genes associated with the given disease from the table of ranked genes for a gene panel design. The processor may be further configured to use the rank scores for the given level to provide the rank scores for a second disease at a second higher level in the disease hierarchy, where the second disease has a hierarchical relationship with the given disease. The processor may be further configured to use the ranked gene information for the given level to provide the ranked gene information for a second disease at a second higher level in the disease hierarchy, where the second disease has a hierarchical relationship with the given disease. The processor may be further configured to use the rank scores for gene-disease associations at a lower level of the disease hierarchy to provide the rank scores for the diseases at a plurality of higher levels of the disease hierarchy where there is a hierarchical relationship between the disease at the lower level with the diseases at the higher levels. The processor may be further configured to use the ranked gene information for the gene-disease associations at a lower level of the disease hierarchy to provide the ranked gene information for the diseases at a plurality of higher levels of the disease hierarchy where there is a hierarchical relationship between the disease at the lower level with the diseases at the higher levels. The processor may be further configured to determine an order of values of the strength parameters for the gene-disease associations at the given level from a highest value to a lowest value and assign an order index to each of the strength parameters based on the order of values, wherein the weight applied to each strength parameter is based on an inverse of its order index. The processor may be further configured to apply a threshold to the rank scores, wherein the threshold has a value in a range of about 0.09-0.10. The processor may be further configured to group the genes into gene clusters based on correlations of the rank scores of the genes associated with the diseases at a level of the disease hierarchy. The processor may be further configured to apply a principal component analysis to the rank scores corresponding to the genes of each gene cluster to determine principal component vectors for the gene clusters.

According to an exemplary embodiment, there is provided a method of selecting genes for a gene panel, including: (1) retrieving gene-disease associations of genes associated with diseases at a given level in a disease hierarchy from a disease association database, the disease association database configured to store disease information for a plurality of diseases and gene information for a plurality of genes, the disease association database including disease associations between diseases in the plurality of diseases and gene-disease associations between the diseases and associated genes in the plurality of genes, wherein the disease associations include the disease hierarchy and the gene-disease associations include a strength parameter for each gene-disease association, the disease association database stored in a memory, wherein the diseases at the given level have hierarchical relationships with a given disease at a higher level in the disease hierarchy; (2) for each gene associated with the diseases at the given level, applying a weight to the strength parameter for each gene-disease association; (3) adding the weighted strength parameters of the gene-disease associations to form a rank score for the each gene associated with the diseases at the given level; and (4) ranking the genes associated with the diseases at the given level based on the rank score to provide ranked gene information associated with the given disease at the higher level for a table of ranked genes. The step of ranking the genes may further include applying a threshold to the rank scores and to rank the genes having rank scores greater than or equal to the threshold. The disease association database may comprise a graph database system having a plurality of nodes and a plurality of edges, wherein an edge is associated with two nodes. A disease node of the plurality of nodes may store the disease information for one of the plurality of diseases. The disease information at the disease node may include a disease identifier. An edge associating two disease nodes may represent a hierarchical relationship of the two diseases. A gene node of the plurality of nodes may store the gene information for one of the plurality of genes. The gene information stored at the gene node may include a gene identifier. A disease node of the plurality of nodes and a gene node of the plurality of nodes are associated by a gene-disease edge, wherein the gene-disease edge stores the strength parameter for the gene-disease association. The disease association database may further include phenotype information for a plurality of phenotypes and phenotype-disease associations between the diseases and associated phenotypes in the plurality of phenotypes. The method may further include a step of responding to a query to the disease association database to provide the gene-disease associations for a graphical display. The method may further include a step of responding to a selection of the given disease by a user to select one or more of the ranked genes associated with the given disease from the table of ranked genes for a gene panel design. The method may further include a step of using the rank scores for the given level to provide the rank scores for a second disease at a second higher level in the disease hierarchy, where the second disease has a hierarchical relationship with the given disease. The method may further include a step of using the ranked gene information for the given level to provide the ranked gene information for a second disease at a second higher level in the disease hierarchy, where the second disease has a hierarchical relationship with the given disease. The method may further include a step of using the ranked gene information for the given level to provide the ranked gene information for a second disease at a second higher level in the disease hierarchy, where the second disease has a hierarchical relationship with the given disease. The method may further include a step of using the rank scores for gene-disease associations at a lower level of the disease hierarchy to provide the rank scores for the diseases at a plurality of higher levels of the disease hierarchy where there is a hierarchical relationship between the disease at the lower level with the diseases at the higher levels. The method may further include a step of using the ranked gene information for the gene-disease associations at a lower level of the disease hierarchy to provide the ranked gene information for the diseases at a plurality of higher levels of the disease hierarchy where there is a hierarchical relationship between the disease at the lower level with the diseases at the higher levels. The method may further include a step of determining an order of values of the strength parameters for the gene-disease associations at the given level from a highest value to a lowest value and assigning an order index to each of the strength parameters based on the order of values, wherein the weight applied to each strength parameter is based on an inverse of its order index. The method may further include a step of applying a threshold to the rank scores, wherein the threshold has a value in a range of about 0.09-0.10. The method may further include a step of grouping the genes into gene clusters based on correlations of the rank scores of the genes associated with the diseases at a level of the disease hierarchy. The method may further include a step of applying a principal component analysis to the rank scores corresponding to the genes of each gene cluster to determine principal component vectors for the gene clusters. According to an exemplary embodiment, there is provided a non-transitory machine-readable storage medium comprising instructions which, when executed by a processor, cause the processor to perform such a method for nucleic acid sequencing or related methods and variants thereof.

A kit comprising a set of primers associated with a set of genes in a gene panel, the set of genes selected for the gene panel by the steps of: (1) retrieving gene-disease associations of genes associated with diseases at a given level in a disease hierarchy from a disease association database, the disease association database configured to store disease information for a plurality of diseases and gene information for a plurality of genes, the disease association database including disease associations between diseases in the plurality of diseases and gene-disease associations between the diseases and associated genes in the plurality of genes, wherein the disease associations include the disease hierarchy and the gene-disease associations include a strength parameter for each gene-disease association, the disease association database stored in a memory, wherein the diseases at the given level have hierarchical relationships with a given disease at a higher level in the disease hierarchy; (2) for each gene associated with the diseases at the given level, applying a weight to the strength parameter for each gene-disease association; (3) adding the weighted strength parameters of the gene-disease associations to form a rank score for the each gene associated with the diseases at the given level; (4) ranking the genes associated with the diseases at the given level based on the rank score to provide ranked gene information associated with the given disease at the higher level for a table of ranked genes; and (5) selecting at least one of the ranked genes from the table of ranked genes for the set of genes in the gene panel. The step of ranking the genes may further include applying a threshold to the rank scores and to rank the genes having rank scores greater than or equal to the threshold. The disease association database may comprise a graph database system having a plurality of nodes and a plurality of edges, wherein an edge is associated with two nodes. A disease node of the plurality of nodes may store the disease information for one of the plurality of diseases. The disease information at the disease node may include a disease identifier. An edge associating two disease nodes may represent a hierarchical relationship of the two diseases. A gene node of the plurality of nodes may store the gene information for one of the plurality of genes. The gene information stored at the gene node may include a gene identifier. A disease node of the plurality of nodes and a gene node of the plurality of nodes are associated by a gene-disease edge, wherein the gene-disease edge stores the strength parameter for the gene-disease association. The disease association database may further include phenotype information for a plurality of phenotypes and phenotype-disease associations between the diseases and associated phenotypes in the plurality of phenotypes. The steps may further include a step of responding to a query to the disease association database to provide the gene-disease associations for a graphical display. The steps may further include a step of responding to a selection of the given disease by a user to select one or more of the ranked genes associated with the given disease from the table of ranked genes for a gene panel design. The steps may further include a step of using the rank scores for the given level to provide the rank scores for a second disease at a second higher level in the disease hierarchy, where the second disease has a hierarchical relationship with the given disease. The steps may further include a step of using the ranked gene information for the given level to provide the ranked gene information for a second disease at a second higher level in the disease hierarchy, where the second disease has a hierarchical relationship with the given disease. The steps may further include a step of using the ranked gene information for the given level to provide the ranked gene information for a second disease at a second higher level in the disease hierarchy, where the second disease has a hierarchical relationship with the given disease. The steps may further include a step of using the rank scores for gene-disease associations at a lower level of the disease hierarchy to provide the rank scores for the diseases at a plurality of higher levels of the disease hierarchy where there is a hierarchical relationship between the disease at the lower level with the diseases at the higher levels. The steps may further include a step of using the ranked gene information for the gene-disease associations at a lower level of the disease hierarchy to provide the ranked gene information for the diseases at a plurality of higher levels of the disease hierarchy where there is a hierarchical relationship between the disease at the lower level with the diseases at the higher levels. The steps may further include a step of determining an order of values of the strength parameters for the gene-disease associations at the given level from a highest value to a lowest value and assigning an order index to each of the strength parameters based on the order of values, wherein the weight applied to each strength parameter is based on an inverse of its order index. The steps may further include a step of applying a threshold to the rank scores, wherein the threshold has a value in a range of about 0.09-0.10. The steps may further include a step of grouping the genes into gene clusters based on correlations of the rank scores of the genes associated with the diseases at a level of the disease hierarchy. The steps may further include a step of applying a principal component analysis to the rank scores corresponding to the genes of each gene cluster to determine principal component vectors for the gene clusters.

According to various exemplary embodiments, one or more features of any one or more of the above-discussed teachings and/or exemplary embodiments may be performed or implemented using appropriately configured and/or programmed hardware and/or software elements. Determining whether an embodiment is implemented using hardware and/or software elements may be based on any number of factors, such as desired computational rate, power levels, heat tolerances, processing cycle budget, input data rates, output data rates, memory resources, data bus speeds, etc., and other design or performance constraints.

Examples of hardware elements may include processors, microprocessors, input(s) and/or output(s) (I/O) device(s) (or peripherals) that are communicatively coupled via a local interface circuit, circuit elements (e.g., transistors, resistors, capacitors, inductors, and so forth), integrated circuits, application specific integrated circuits (ASIC), programmable logic devices (PLD), digital signal processors (DSP), field programmable gate array (FPGA), logic gates, registers, semiconductor device, chips, microchips, chip sets, and so forth. The local interface may include, for example, one or more buses or other wired or wireless connections, controllers, buffers (caches), drivers, repeaters and receivers, etc., to allow appropriate communications between hardware components. A processor is a hardware device for executing software, particularly software stored in memory. The processor can be any custom made or commercially available processor, a central processing unit (CPU), an auxiliary processor among several processors associated with the computer, a semiconductor based microprocessor (e.g., in the form of a microchip or chip set), a macroprocessor, or generally any device for executing software instructions. A processor can also represent a distributed processing architecture. The I/O devices can include input devices, for example, a keyboard, a mouse, a scanner, a microphone, a touch screen, an interface for various medical devices and/or laboratory instruments, a bar code reader, a stylus, a laser reader, a radio-frequency device reader, etc. Furthermore, the I/O devices also can include output devices, for example, a printer, a bar code printer, a display, etc. Finally, the I/O devices further can include devices that communicate as both inputs and outputs, for example, a modulator/demodulator (modem; for accessing another device, system, or network), a radio frequency (RF) or other transceiver, a telephonic interface, a bridge, a router, etc.

Examples of software may include software components, programs, applications, computer programs, application programs, system programs, machine programs, operating system software, middleware, firmware, software modules, routines, subroutines, functions, methods, procedures, software interfaces, application program interfaces (API), instruction sets, computing code, computer code, code segments, computer code segments, words, values, symbols, or any combination thereof. A software in memory may include one or more separate programs, which may include ordered listings of executable instructions for implementing logical functions. The software in memory may include a system for identifying data streams in accordance with the present teachings and any suitable custom made or commercially available operating system (O/S), which may control the execution of other computer programs such as the system, and provides scheduling, input-output control, file and data management, memory management, communication control, etc.

According to various exemplary embodiments, one or more features of any one or more of the above-discussed teachings and/or exemplary embodiments may be performed or implemented using appropriately configured and/or programmed non-transitory machine-readable medium or article that may store an instruction or a set of instructions that, if executed by a machine, may cause the machine to perform a method and/or operations in accordance with the exemplary embodiments. Such a machine may include, for example, any suitable processing platform, computing platform, computing device, processing device, computing system, processing system, computer, processor, scientific or laboratory instrument, etc., and may be implemented using any suitable combination of hardware and/or software. The machine-readable medium or article may include, for example, any suitable type of memory unit, memory device, memory article, memory medium, storage device, storage article, storage medium and/or storage unit, for example, memory, removable or non-removable media, erasable or non-erasable media, writeable or re-writeable media, digital or analog media, hard disk, floppy disk, read-only memory compact disc (CD-ROM), recordable compact disc (CD-R), rewriteable compact disc (CD-RW), optical disk, magnetic media, magneto-optical media, removable memory cards or disks, various types of Digital Versatile Disc (DVD), a tape, a cassette, etc., including any medium suitable for use in a computer. Memory can include any one or a combination of volatile memory elements (e.g., random access memory (RAM, such as DRAM, SRAM, SDRAM, etc.)) and nonvolatile memory elements (e.g., ROM, EPROM, EEROM, Flash memory, hard drive, tape, CDROM, etc.). Moreover, memory can incorporate electronic, magnetic, optical, and/or other types of storage media. Memory can have a distributed architecture where various components are situated remote from one another, but are still accessed by the processor. The instructions may include any suitable type of code, such as source code, compiled code, interpreted code, executable code, static code, dynamic code, encrypted code, etc., implemented using any suitable high-level, low-level, object-oriented, visual, compiled and/or interpreted programming language.

According to various exemplary embodiments, one or more features of any one or more of the above-discussed teachings and/or exemplary embodiments may be performed or implemented at least partly using a distributed, clustered, remote, or cloud computing resource.

According to various exemplary embodiments, one or more features of any one or more of the above-discussed teachings and/or exemplary embodiments may be performed or implemented using a source program, executable program (object code), script, or any other entity comprising a set of instructions to be performed. When a source program, the program can be translated via a compiler, assembler, interpreter, etc., which may or may not be included within the memory, so as to operate properly in connection with the O/S. The instructions may be written using (a) an object oriented programming language, which has classes of data and methods, or (b) a procedural programming language, which has routines, subroutines, and/or functions, which may include, for example, C, C++, R, Pascal, Basic, Fortran, Cobol, Perl, Java, and Ada.

According to various exemplary embodiments, one or more of the above-discussed exemplary embodiments may include transmitting, displaying, storing, printing or outputting to a user interface device, a computer readable storage medium, a local computer system or a remote computer system, information related to any information, signal, data, and/or intermediate or final results that may have been generated, accessed, or used by such exemplary embodiments. Such transmitted, displayed, stored, printed or outputted information can take the form of searchable and/or filterable lists of runs and reports, pictures, tables, charts, graphs, spreadsheets, correlations, sequences, and combinations thereof, for example.

While preferred embodiments of the present invention have been shown and described herein, it will be obvious to those skilled in the art that such embodiments are provided by way of example only. Numerous variations, changes, and substitutions will now occur to those skilled in the art. It should be understood that various alternatives to the embodiments of the invention described herein may be employed in practicing the invention. It is intended that the following claims define the scope of the invention.

**Claims**

1. Non-transitory machine-readable medium or article that stores an instruction or a set of instructions that, if executed by a machine including a processing platform, computing platform, computing device, processing device, computing system, processing system, computer, processor, scientific or laboratory instrument, causes the machine to perform a method and/or operations comprising the steps of:

   retrieving gene-disease associations of genes associated with diseases at a given level in a disease hierarchy from a disease association database (110), the disease association database configured to store disease information for a plurality of diseases (341, 342, 343, 344, 345, 346, 420, 441, 442, 443, 445, 447) and gene information for a plurality of genes (360), the disease association database including disease associations between the diseases in the plurality of diseases and gene-disease associations (352, 354) between the diseases and associated genes in the plurality of genes, wherein the disease associations include the disease hierarchy and the gene-disease associations include a strength parameter for each gene-disease association, the disease association database stored in a memory, wherein the diseases at the given level have hierarchical relationships with a disease of interest (420) at a higher level in the disease hierarchy;
   for each gene associated with the diseases at the given level, applying a weight to the strength parameter for each gene-disease association to form a weighted strength parameter;
   adding the weighted strength parameters of the gene-disease associations to form a rank score for each gene associated with the diseases at the given level;
   ranking the genes associated with the diseases at the given level based on the rank score to provide ranked gene information associated with the disease of interest at the higher level for a table of ranked genes;
   linking the selected rank scores and ranked gene information to the disease of interest, and
   selecting at least one of the ranked genes from the table of ranked genes for the set of genes in the gene panel in response to a selection of the disease of interest by a user.

2. A computer-implemented method of selecting genes for a gene panel, comprising:

   retrieving gene-disease associations of genes associated with diseases at a given level in a disease hierarchy from a disease association database (110), the disease association database configured to store disease information for a plurality of diseases (341, 342, 343, 344, 345, 346, 420, 441, 442, 443, 445, 447) and gene information for a plurality of genes (360), the disease association database including disease associations between the diseases in the plurality of diseases and gene-disease associations (352, 354) between the diseases and associated genes in the plurality of genes, wherein the disease associations include the disease hierarchy and the gene-disease associations include a strength parameter for each gene-disease association, the disease association database stored in a memory, wherein the diseases at the given level have hierarchical relationships with a disease of interest (420) at a higher level in the disease hierarchy;
   for each gene associated with the diseases at the given level, applying a weight to the strength parameter for each gene-disease association to form a weighted strength parameter;
   adding the weighted strength parameters of the gene-disease associations to form a rank score for each gene associated with the diseases at the given level; and
   ranking the genes associated with the diseases at the given level based on the rank score to provide ranked gene information associated with the disease of interest at the higher level for a table of ranked genes;
   linking the selected rank scores and ranked gene information to the disease of interest,
   and selecting at least one of the ranked genes from the table of ranked genes for the set of genes in the gene panel in response to a selection of the disease of interest by a user.

3. The method of claim 2, wherein for the ranking step, a threshold is applied to the rank scores and to rank the genes having rank scores greater than or equal to the threshold.

4. The method of claim 2, wherein the disease association database comprises a graph database system having a plurality of nodes and a plurality of edges, wherein an edge is associated with two nodes.

5. The method of claim 4, wherein a disease node of the plurality of nodes stores the disease information for one of the plurality of diseases.

6. The method of claim 5, wherein the disease information at the disease node includes a disease identifier.

7. The method of claim 5, wherein an edge associating two disease nodes represents a hierarchical relationship of the two diseases.

8. The method of claim 4, wherein a gene node of the plurality of nodes stores the gene information for one of the plurality of genes.

9. The method of claim 8, wherein the gene information stored at the gene node includes a gene identifier.

10. The method of claim 4, wherein a disease node of the plurality of nodes and a gene node of the plurality of nodes are associated by a gene-disease edge, wherein the gene-disease edge stores the strength parameter for the gene-disease association.

11. The method of claim 2, wherein the disease association database further includes phenotype information for a plurality of phenotypes and phenotype-disease associations between the diseases and associated phenotypes in the plurality of phenotypes.

12. The method of claim 2, further including: responding to a selection of the given disease by a user to select one or more of the ranked genes associated with the given disease from the table of ranked genes for a gene panel design.

13. The method of claim 2, further including: using the rank scores for gene-disease associations at a lower level of the disease hierarchy providing the rank scores for the diseases at a plurality of higher levels of the disease hierarchy where there is a hierarchical relationship between the disease at the lower level with the diseases at the higher levels.

14. The method of claim 2, further including: using the ranked gene information for the gene-disease associations at a lower level of the disease hierarchy to provide the ranked gene information for the diseases at a plurality of higher levels of the disease hierarchy where there is a hierarchical relationship between the disease at the lower level with the diseases at the higher levels.

15. The method of claim 2, further including:

   determining an order of values of the strength parameters for the gene- disease associations at the given level from a highest value to a lowest value, and
   assigning an order index to each of the strength parameters based on the order of values, wherein the weight applied to each strength parameter is based on an inverse of its order index.

16. Combination of a kit comprising a set of primers associated with a set of genes in a gene panel, the set of genes selected for the gene panel by the method of any of the claims 2 - 15, and a non-transitory machine-readable medium or article according to claim 1.

**Patentansprüche**

1. Nichtflüchtiges maschinenlesbares Medium oder Erzeugnis, das eine Anweisung oder einen Satz von Anweisungen speichert, die, wenn sie durch eine Maschine ausgeführt werden, die eine Verarbeitungsplattform, eine Rechenplattform, eine Rechenvorrichtung, eine Verarbeitungsvorrichtung, ein Rechensystem, ein Verarbeitungssystem, einen Computer, einen Prozessor, ein wissenschaftliches oder ein Laborinstrument einschließt, die Maschine veranlassen, ein Verfahren und/oder Vorgänge durchzuführen, umfassend die Schritte:

   Abrufen von Genkrankheitszuordnungen von Genen, die mit Krankheiten auf einer gegebenen Stufe in einer Krankheitshierarchie zugeordnet sind, von einer Krankheitszuordnungsdatenbank (110), wobei die Krankheitszuordnungsdatenbank konfiguriert ist, um Krankheitsinformationen für eine Vielzahl von Krankheiten (341, 342, 343, 344, 345, 346, 420, 441, 442, 443, 445, 447) und Geninformationen für eine Vielzahl von Genen (360) zu speichern, wobei die Krankheitszuordnungsdatenbank Krankheitszuordnungen zwischen den Krankheiten in der Vielzahl von Krankheiten und Genkrankheitszuordnungen (352, 354) zwischen den Krankheiten und zugeordneten Genen in der Vielzahl von Genen einschließt, wobei die Krankheitszuordnungen die Krankheitshierarchie einschließen und die Genkrankheitszuordnungen einen Stärkeparameter für jede Genkrankheitszuordnung einschließen, die Krankheitszuordnungsdatenbank in einem Speicher gespeichert ist, wobei die Krankheiten auf der gegebenen Stufe hierarchische Beziehungen mit einer Krankheit von Interesse (420) auf einer höheren Stufe in der Krankheitshierarchie aufweisen;

für jedes Gen, das den Krankheiten auf der gegebenen Stufe zugeordnet ist, Anwenden eines Gewichts auf den Stärkeparameter für jede Genkrankheitszuordnung, um einen gewichteten Stärkeparameter auszubilden;

Hinzufügen der gewichteten Stärkeparameter der Genkrankheitszuordnungen, um eine Rangbewertung für jedes Gen auszubilden, das den Krankheiten auf der gegebenen Stufe zugeordnet ist;

Bewerten der Gene, die den Krankheiten auf der gegebenen Stufe basierend auf der Rangbewertung zugeordnet sind, mit einen Rang, um rangbewertete Geninformationen bereitzustellen, die der Krankheit von Interesse auf der höheren Stufe für eine Tabelle von rang bewerteten Genen zugeordnet sind;

Verknüpfen der ausgewählten Rangbewertungen und der rangbewerteten Geninformationen zu der Krankheit von Interesse und

Auswählen mindestens eines der rang bewerteten Gene aus der Tabelle von rang bewerteten Genen für den Satz von Genen in dem Genpanel als Reaktion auf eine Auswahl der Krankheit von Interesse durch einen Benutzer.

2. Computerimplementiertes Verfahren zum Auswählen von Genen für ein Genpanel, umfassend:

Abrufen von Genkrankheitszuordnungen von Genen, die mit Krankheiten auf einer gegebenen Stufe in einer Krankheitshierarchie zugeordnet sind, von einer Krankheitszuordnungsdatenbank (110), wobei die Krankheitszuordnungsdatenbank konfiguriert ist, um Krankheitsinformationen für eine Vielzahl von Krankheiten (341, 342, 343, 344, 345, 346, 420, 441, 442, 443, 445, 447) und Geninformationen für eine Vielzahl von Genen (360) zu speichern, wobei die Krankheitszuordnungsdatenbank Krankheitszuordnungen zwischen den Krankheiten in der Vielzahl von Krankheiten und Genkrankheitszuordnungen (352, 354) zwischen den Krankheiten und zugeordneten Genen in der Vielzahl von Genen einschließt, wobei die Krankheitszuordnungen die Krankheitshierarchie einschließen und die Genkrankheitszuordnungen einen Stärkeparameter für jede Genkrankheitszuordnung einschließen, die Krankheitszuordnungsdatenbank in einem Speicher gespeichert ist, wobei die Krankheiten auf der gegebenen Stufe hierarchische Beziehungen mit einer Krankheit von Interesse (420) auf einer höheren Stufe in der Krankheitshierarchie aufweisen;

für jedes Gen, das den Krankheiten auf der gegebenen Stufe zugeordnet ist, Anwenden eines Gewichts auf den Stärkeparameter für jede Genkrankheitszuordnung, um einen gewichteten Festigkeitsparameter auszubilden;

Hinzufügen der gewichteten Stärkeparameter der Genkrankheitszuordnungen, um eine Rangbewertung für jedes Gen auszubilden, das den Krankheiten auf der gegebenen Stufe zugeordnet ist; und

Bewerten der Gene, die den Krankheiten auf der gegebenen Stufe basierend auf der Rangbewertung zugeordnet sind, mit einem Rang, um rangbewertete Geninformationen bereitzustellen, die der Krankheit von Interesse auf der höheren Stufe für eine Tabelle von rang bewerteten Genen zugeordnet sind;

Verknüpfen der ausgewählten Rangbewertungen und der rangbewerteten Geninformationen zu der Krankheit von Interesse,

und Auswählen mindestens eines der rangbewerten Gene aus der Tabelle von rangbewerten Genen für den Satz von Genen in dem Genpanel als Reaktion auf eine Auswahl der Krankheit von Interesse durch einen Benutzer.

3. Verfahren nach Anspruch 2, wobei für den Schritt der Bewertung mit einem Rang, ein Schwellenwert auf die Rangbewertungen angewendet wird und um die Gene zu mit einem Rang zu bewerten, die Rangbewertungen größer als oder gleich dem Schwellenwert aufweisen.

4. Verfahren nach Anspruch 2, wobei die Krankheitszuordnungsdatenbank ein graphisches Datenbanksystem umfasst, das eine Vielzahl von Knoten und eine Vielzahl von Kanten aufweist, wobei eine Kante zwei Knoten zugeordnet ist.

5. Verfahren nach Anspruch 4, wobei ein Krankheitsknoten der Vielzahl von Knoten die Krankheitsinformationen für eine der mehreren Krankheiten speichert.

6. Verfahren nach Anspruch 5, wobei die Krankheitsinformationen an dem Krankheitsknoten eine Krankheitskennung einschließen.

7. Verfahren nach Anspruch 5, wobei eine Kante, der zwei Krankheitsknoten zuordnet, eine hierarchische Beziehung der zwei Krankheiten darstellt.

8. Verfahren nach Anspruch 4, wobei ein Genknoten der Vielzahl von Knoten die Geninformationen für eines der Vielzahl von Genen speichert.

9. Verfahren nach Anspruch 8, wobei die Geninformationen, die an dem Genknoten gespeichert sind, eine Genkennung einschließen.

10. Verfahren nach Anspruch 4, wobei ein Krankheitsknoten der Vielzahl von Knoten und ein Genknoten der Vielzahl von Knoten durch eine Genkrankheitskante zugeordnet sind, wobei die Genkrankheitskante den Festigkeitsparameter für die Generkrankungszuordnung speichert.

11. Verfahren nach Anspruch 2, wobei die Krankheitszuordnungsdatenbank ferner Phänotypinformationen für eine Vielzahl von Phänotypen und Phänotypkrankheit zwischen den Krankheiten und zugeordneten Phänotypen in der Vielzahl von Phänotypen einschließt.

12. Verfahren nach Anspruch 2, das ferner einschließt: Reagieren auf eine Auswahl der gegebenen Krankheit durch einen Benutzer, um eines oder mehrere der rang bewerteten Gene, die der gegebenen Krankheit zugeordnet sind, aus der Tabelle von rang bewerteten Genen für ein Genpaneldesign auszuwählen.

13. Verfahren nach Anspruch 2, das ferner einschließt: Verwenden der Rangbewertungen für Genkrankheitszuordnungen auf einer niedrigeren Stufe der Krankheitshierarchie, die Rangbewertungen für die Krankheiten bei einer Vielzahl von höheren Stufen der Krankheitshierarchie bereitstellt, wobei es eine hierarchische Beziehung zwischen der Krankheit auf der niedrigeren Stufe mit den Krankheiten auf den höheren Stufe gibt.

14. Verfahren nach Anspruch 2, das ferner einschließt: Verwenden der rangbewerteten Geninformationen für die Genkrankheitszuordnungen auf einer niedrigeren Stufe der Krankheitshierarchie, um die rangbewerteten Geninformationen für die Krankheiten bei einer Vielzahl von höheren Stufen der Krankheitshierarchie bereitzustellen, wobei es eine hierarchische Beziehung zwischen der Krankheit auf der niedrigeren Stufe mit den Krankheiten auf den höheren Stufe gibt.

15. Verfahren nach Anspruch 2, das ferner einschließt:

Bestimmen einer Reihenfolge von Werten der Festigkeitsparameter für die Genkrankheitszuordnungen auf der gegebenen Stufe von einem höchsten Wert zu einem niedrigsten Wert, und
Zuweisen eines Ordnungsindexes zu jedem der Festigkeitsparameter basierend auf der Reihenfolge der Werte, wobei das Gewicht, das auf jeden Festigkeitsparameter angewendet wird, auf einer Inversen seines Ordnungsindex basiert.

16. Kombination eines Kits, umfassend einen Satz von Primern, die einem Satz von Genen in einem Genpanel zugeordnet sind, wobei der Satz von Genen für das Genpanel durch das Verfahren nach einem der Ansprüche 2 bis 15 und ein nichtflüchtiges maschinenlesbares Medium oder Erzeugnis nach Anspruch 1 ausgewählt ist.

**Revendications**

1. Article ou support lisible par machine non transitoire qui stocke une instruction ou un ensemble d'instructions qui, si elles sont exécutées par une machine comportant une plateforme de traitement, une plateforme informatique, un dispositif informatique, un dispositif de traitement, un système informatique, un système de traitement, un ordinateur, un processeur, un instrument scientifique ou de laboratoire, amènent la machine à effectuer un procédé et/ou des opérations comprenant les étapes consistant à :

récupérer des associations gène-maladie de gènes associés à des maladies à un niveau donné dans une hiérarchie de maladies à partir d'une base de données d'association de maladies (110), la base de données d'association de maladies étant configurée pour stocker des informations de maladie pour une pluralité de maladies (341, 342, 343, 344, 345, 346, 420, 441, 442, 443, 445, 447) et des informations de gène pour une pluralité de gènes (360), la base de données d'association de maladies comportant des associations de maladies entre les maladies dans la pluralité de maladies et des associations gène-maladie (352, 354) entre les maladies et gènes associés dans la pluralité de gènes, dans lequel les associations de maladies comportent la hiérarchie de maladies et les associations gène-maladie comportent un paramètre de force pour chaque association gène-maladie, la base de données d'association de maladies étant stockée dans une mémoire, dans lequel les maladies au niveau donné ont des relations hiérarchiques avec une maladie d'intérêt (420) à un niveau supérieur dans la hiérarchie de maladies ;

pour chaque gène associé aux maladies au niveau donné, appliquer un poids au paramètre de force pour chaque association gène-maladie pour former un paramètre de force pondéré ;

ajouter les paramètres de force pondérés des associations gène-maladie pour former un score de classement pour chaque gène associé aux maladies au niveau donné ;

classer les gènes associés aux maladies au niveau donné sur la base du score de classement pour fournir des informations de gènes classés associées à la maladie d'intérêt au niveau supérieur pour un tableau de gènes classés ;

relier les scores de classement sélectionnés et les informations de gènes classés à la maladie d'intérêt, et sélectionner au moins un des gènes classés à partir du tableau de gènes classés pour l'ensemble de gènes dans le panel de gènes en réponse à une sélection de la maladie d'intérêt par un utilisateur.

2. Procédé mis en oeuvre par ordinateur de sélection de gènes pour un panel de gènes, comprenant :

la récupération d'associations gène-maladie de gènes associés à des maladies à un niveau donné dans une hiérarchie de maladies à partir d'une base de données d'association de maladies (110), la base de données d'association de maladies étant configurée pour stocker des informations de maladie pour une pluralité de maladies (341, 342, 343, 344, 345, 346, 420, 441, 442, 443, 445, 447) et des informations de gène pour une pluralité de gènes (360), la base de données d'association de maladies comportant des associations de maladies entre les maladies dans la pluralité de maladies et des associations gène-maladie (352, 354) entre les maladies et gènes associés dans la pluralité de gènes, dans lequel les associations de maladies comportent la hiérarchie de maladies et les associations gène-maladie comportent un paramètre de force pour chaque association gène-maladie, la base de données d'association de maladies étant stockée dans une mémoire, dans lequel les maladies au niveau donné ont des relations hiérarchiques avec une maladie d'intérêt (420) à un niveau supérieur dans la hiérarchie de maladies ;

pour chaque gène associé aux maladies au niveau donné, l'application d'un poids au paramètre de force pour chaque association gène-maladie pour former un paramètre de force pondéré ;

l'ajout des paramètres de force pondérés des associations gène-maladie pour former un score de classement pour chaque gène associé aux maladies au niveau donné ; et

le classement des gènes associés aux maladies au niveau donné sur la base du score de classement pour fournir des informations de gènes classés associées à la maladie d'intérêt au niveau supérieur pour un tableau de gènes classés ;

la liaison des scores de classement sélectionnés et des informations de gènes classés à la maladie d'intérêt, et la sélection d'au moins l'un des gènes classés à partir du tableau de gènes classés pour l'ensemble de gènes dans le panel de gènes en réponse à une sélection de la maladie d'intérêt par un utilisateur.

3. Procédé selon la revendication 2, dans lequel pour l'étape de classement, un seuil est appliqué aux scores de classement et pour classer les gènes ayant des scores de classement supérieurs ou égaux au seuil.

4. Procédé selon la revendication 2, dans lequel la base de données d'association de maladies comprend un système de base de données de graphes ayant une pluralité de noeuds et une pluralité de bords, dans lequel un bord est associé à deux noeuds.

5. Procédé selon la revendication 4, dans lequel un noeud de maladie de la pluralité de noeuds stocke les informations de maladie pour l'une de la pluralité de maladies.

6. Procédé selon la revendication 5, dans lequel les informations de maladie au niveau du noeud de maladie comportent un identifiant de maladie.

7. Procédé selon la revendication 5, dans lequel un bord associant deux noeuds de maladie représente une relation hiérarchique des deux maladies.

8. Procédé selon la revendication 4, dans lequel un noeud de gène de la pluralité de noeuds stocke les informations de gène pour l'un de la pluralité de gènes.

9. Procédé selon la revendication 8, dans lequel les informations de gène stockées au niveau du noeud de gène comportent un identifiant de gène.

**10.** Procédé selon la revendication 4, dans lequel un noeud de maladie de la pluralité de noeuds et un noeud de gène de la pluralité de noeuds sont associés par un bord gène-maladie, dans lequel le bord gène-maladie stocke le paramètre de force pour l'association gène-maladie.

**11.** Procédé selon la revendication 2, dans lequel la base de données d'association de maladies comporte en outre des informations de phénotype pour une pluralité de phénotypes et des associations phénotype-maladie entre les maladies et phénotypes associés dans la pluralité de phénotypes.

**12.** Procédé selon la revendication 2, comportant en outre : la réponse à une sélection de la maladie donnée par un utilisateur pour sélectionner un ou plusieurs des gènes classés associés à la maladie donnée à partir du tableau de gènes classés pour une conception de panel de gènes.

**13.** Procédé selon la revendication 2, comportant en outre : l'utilisation des scores de classement pour des associations gène-maladie à un niveau inférieur de la hiérarchie de maladies fournissant les scores de classement pour les maladies à une pluralité de niveaux supérieurs de la hiérarchie de maladies où il existe une relation hiérarchique entre la maladie au niveau inférieur avec les maladies aux niveaux supérieurs.

**14.** Procédé selon la revendication 2, comportant en outre : l'utilisation des informations de gènes classés pour les associations gène-maladie à un niveau inférieur de la hiérarchie de maladies pour fournir les informations de gènes classés pour les maladies à une pluralité de niveaux supérieurs de la hiérarchie de maladies où il existe une relation hiérarchique entre la maladie au niveau inférieur avec les maladies aux niveaux supérieurs.

**15.** Procédé selon la revendication 2, comportant en outre :

la détermination d'un ordre de valeurs des paramètres de force pour les associations gène-maladie au niveau donné d'une valeur la plus élevée à une valeur la plus faible, et
l'attribution d'un indice d'ordre à chacun des paramètres de force sur la base de l'ordre de valeurs, dans lequel le poids appliqué à chaque paramètre de force est basé sur un inverse de son indice d'ordre.

**16.** Combinaison d'une trousse comprenant un ensemble d'amorces associées à un ensemble de gènes dans un panel de gènes, l'ensemble de gènes étant choisi pour le panel de gènes par le procédé selon l'une quelconque des revendications 2 à 15, et un article ou support lisible par machine non transitoire selon la revendication 1.

FIG. 1

EP 3 465 506 B1

FIG. 2

FIG. 3

FIG. 4

FIG. 5

FIG. 6

Disease hierarchy

| Level 4 | MeSH ID | Gene Symbol | Score | Gene Rank |
|---------|---------|-------------|-------|-----------|
| | | | | 1 |
| | | | | 2 |
| | | | | 3 |

| Level 3 | MeSH ID | Gene Symbol | Score | Gene Rank |
|---------|---------|-------------|-------|-----------|
| | | | | 1 |
| | | | | 2 |
| | | | | 3 |
| | | | | 4 |
| | | | | 5 |
| | | | | 6 |
| | | | | 7 |
| | | | | 8 |
| | | | | 9 |
| | | | | 10 |
| | | | | 11 |
| | | | | 12 |
| | | | | 13 |
| | | | | 14 |
| | | | | 15 |

| Level 2 | MeSH ID | Gene Symbol | Score | Gene Rank |
|---------|---------|-------------|-------|-----------|
| | | | | 1 |
| | | | | 2 |
| | | | | 3 |
| | | | | 4 |
| | | | | 5 |
| | | | | 6 |
| | | | | 7 |
| | | | | 8 |
| | | | | 9 |
| | | | | 10 |
| | | | | 11 |
| | | | | 12 |
| | | | | 13 |
| | | | | 14 |
| | | | | 15 |

| Level 1 | MeSH ID | Gene Symbol | Score | Gene Rank |
|---------|---------|-------------|-------|-----------|
| | D001523 | PRNP | 0.96 | 1 |
| | D001523 | MAPT | 0.88 | 2 |
| | D001523 | APP | 0.81 | 3 |
| | D001523 | SLC6A4 | 0.81 | 4 |
| | D001523 | BDNF | 0.77 | 5 |
| | D001523 | APOE | 0.73 | 6 |
| | D001523 | PSEN1 | 0.72 | 7 |
| | D001523 | HTT | 0.71 | 8 |
| | D001523 | DRD2 | 0.67 | 9 |
| | D001523 | HTR2A | 0.67 | 10 |
| | D001523 | COMT | 0.67 | 11 |
| | D001523 | SLC6A3 | 0.66 | 12 |
| | D001523 | DRD4 | 0.59 | 13 |
| | D001523 | RELN | 0.58 | 14 |
| | D001523 | SNCA | 0.56 | 15 |

FIG. 7

EP 3 465 506 B1

FIG. 8

820

Extract gene-disease association scores
from DAD

840

Construct a gene-disease association network

860

Identify gene clusters (modules)

880

Annotate gene modules by disease

FIG. 9

| Module Number | Gene Count | Annotation |
|---|---|---|
| 1 | 530 | Nervous System Diseases |
| 2 | 321 | Nutritional and Metabolic Diseases |
| 3 | 307 | Cardiovascular Diseases |
| 4 | 280 | Digestive System Diseases |
| 5 | 253 | Eye Diseases |
| 6 | 250 | Skin and Tissue Connective Diseases |
| 7 | 229 | Male and Female Urogenital Diseases |
| 8 | 205 | Musculoskeletal Diseases |
| 9 | 164 | Nervous System Diseases; Nutritional and Metabolic Diseases |
| 10 | 150 | Hemic and Lymphatic Diseases |
| 11 | 140 | Musculoskeletal Diseases; Nervous System Diseases |
| 12 | 137 | Neoplasms |
| 13 | 129 | Respiratory Tract Diseases |
| 14 | 111 | Otorhinolaryngologic Diseases; Nervous System Diseases |
| 15 | 90 | Male Urogenital Diseases |
| 16 | 87 | Immune; Male Urogenital Diseases; Female Urogenital Diseases and Pregnancy Complications |
| 17 | 76 | Stomatognathic Diseases |
| 18 | 69 | Hemic and Lymphatic Diseases; Immune System Diseases |
| 19 | 67 | Female Urogenital Diseases and Pregnancy Complications; Endocrine Diseases |
| 20 | 63 | Female Urogenital Diseases and Pregnancy Complications |
| 21 | 61 | Musculoskeletal Diseases; Skin and Connective Tissue Diseases |
| 22 | 60 | Musculoskelatal Diseases; Stomatognathic Diseases |
| 23 | 55 | Female and Male Urogenital Diseases; Nutritional and Metabolic Diseases |
| 24 | 55 | Nutritional and Metabolic Diseases; Endocrine System Diseases |
| 25 | 36 | Musculoskeletal Diseases; Cardiovascular Diseases |
| 26 | 36 | Immune System Diseases |
| 27 | 35 | Endrocrine System Diseases |
| 28 | 34 | Immune System Diseases; Skin and Connective Tissue Diseases |

## FIG. 10

FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5210015 A, Gelfand
- US 5399491 A, Kacian
- US 4683202 A, Mullis
- US 4683195 A, Mullis
- US 4965188 A
- US 4800159 A
- US 5854033 A, Lizardi
- US 6174670 B, Wittwer
- US 20090137404 A, Drmanac
- US 20100295819 A, Leamon

### Non-patent literature cited in the description

- **BODENREIDER.** The Unified Medical Language System (UMLS): integrating biomedical terminology. *Nucleic Acids Research,* 2004, vol. 32, D267-D270
- **PIÑERO et al.** DisGeNET: a discovery platform for the dynamical exploration of human diseases and their genes. *Database,* 2015, vol. 2015, 1-17
- **LANGFELDER et al.** WGCNA: an R package for weighted correlation network analysis. *BMC Bioinformatics,* 2008, vol. 9 (559), 1-13